# EUROPEAN PATENT APPLICATION

(11) **EP 4 310 849 A1**
(43) Date of publication of application: **24.01.2024**
(21) Application number: 22771496.1
(22) Date of filing: 17.03.2022
(51) Int. Cl.: G16H 20/00, A61B 5/00

(54) **INFORMATION PROCESSING DEVICE, INFORMATION PROCESSING SYSTEM, INFORMATION PROCESSING METHOD, AND PROGRAM**

(30) Priority: 18.03.2021 JP 2021044647
(71) Applicant: Mitsubishi Tanabe Pharma Corporation, Osaka 541-8505 (JP)
(72) Inventor: USHIROGAWA Yoshiteru, Osaka-shi, Osaka 541-8505 (JP); TAKAHASHI Fumihiro, Osaka-shi, Osaka 541-8505 (JP)
(74) Representative: J A Kemp LLP
(86) International application number: PCT/JP2022/012165
(87) International publication number: WO 2022/196751

(57) **Abstract**

[Summary]

[Problem] An information processing device capable of predicting a timing of appearing a symptom related to photosensitivity in a patient with photosensitivity, is provided.

[Solution] An information processing device comprising:
an acquisition unit acquires a reference information set including one or more reference information selected from below (a) to (1), in addition an environmental data; and
a generation unit generates, based on the environmental data and the reference information set, a provision information for providing to a predetermined terminal;
(a) a daylight hour until appearing a photosensitivity related symptom
(b) a timing of the photosensitivity related symptom
(c) a grade of the photosensitivity related symptom
(d) PGIC (Patient Global Impression of Change) score
(e) a skin pigment information
(f) a melanin density
(g) a quantity of a protoporphyrin in a blood
(h) a type of a medicine
(i) a prescription quantity of a medicine
(j) Fitzpatrick skin type
(k) PGIS score (Patient Global Impression Severity)
(l) a data acquired by PROMIS (Patient-Reported Outcomes Measurement Information System).

## Description

### [Field of the Invention]

The present invention relates to an information processing device, an information processing system, an information processing method, and program.

### [Background of the Invention]

A photosensitivity is a general term for skin diseases that are caused or exacerbated by exposure to a sunlight or an ultraviolet ray, and appears a photosensitivity related symptom by a level of a light irradiation that a healthy people do not react.

There are few fundamental treatments for the photosensitivity, a main method that avoiding or alleviating to appear the symptom is avoiding to expose the sunlight to a skin. For avoiding to expose a sunlight to the skin, for instance, there is a technique of notifying a weather information to a user (see, e. g., Patent Literature 1, etc.).

### [Prior Art List]

### [Patent Literature]

Patent Literature 1: Japanese Unexamined Patent Publication No.2020-77197

### [Summary of the Invention]

### [Technical Problem]

A patient suffering from the photosensitivity tend to avoid going out during the day by fear of appearing the photosensitivity related symptom, by knowing a timing of the appearing the photosensitivity related symptom in advance, the patient has desire to increase QOL (Quality of Life).

An object of a present invention is providing an information processing device capable of predicting the timing of appearing the photosensitivity related symptom to a photosensitivity patient.

### [TECHNICAL SOLUTION]

The main invention of the present invention for solving above a problem is
an information processing device comprising:
an acquisition unit acquires a reference information set including one or more reference information selected from below (a) to (1), in addition an environmental data; and
a generation unit generates, based on the reference information set and the environmental data, a provision information for providing to a predetermined terminal;
   (a) a daylight hour until appearing a photosensitivity related symptom
   (b) a timing of the photosensitivity related symptom
   (c) a grade of the photosensitivity related symptom
   (d) PGIC (Patient Global Impression of Change) score
   (e) a skin pigment information
   (f) a melanin density
   (g) a quantity of a protoporphyrin in a blood
   (h) a type of a medicine
   (i) a prescription quantity of a medicine
   (j) Fitzpatrick skin type
   (k) PGIS score (Patient Global Impression Severity)
   (l) a data acquired by PROMIS (Patient-Reported Outcomes Measurement Information System)

In addition, problems and their solutions disclosed by a present application will be clarified in a section of an embodiment of an invention and drawings.

### [ADVANTAGEOUS EFFECTS]

According to the present invention, it is possible to provide the information processing device capable of predicting the timing of appearing the photosensitivity related symptom to the photosensitivity patient.

### [Brief description of The Drawings]

FIG. 1 is a diagram showing a configuration instance of an information processing system including an information processing device.
Fig. 2 is a diagram showing a hardware configuration instance of an information processing device.
Fig. 3 is a diagram showing a software configuration instance of an information processing device.
Fig. 4 is a diagram showing a configuration instance of a patient information stored in a patient information storage unit.
Fig. 5 is a diagram showing a configuration instance of an environmental data stored in an environmental data storage unit.
Fig. 6 is a diagram showing a configuration instance of a doctor input information stored in a doctor input information storage unit.
Fig. 7 is a diagram showing a configuration instance of a provision information stored in a provision information storage unit.
FIG. 8 is a diagram showing a flow of processing executed in the information processing device.

### [Detailed description of The Preferred Embodiment]

Contents of the embodiments of the present invention will be listed and explained. The present invention has, for instance, below configuration.

### [Item 1]

An information processing device comprising:
an acquisition unit acquires a reference information set including one or more reference information selected from below (a) to (1), in addition an environmental data; and
a generation unit generates, based on the reference information set and the environmental data, a provision information for providing to a predetermined terminal;
   (a) a daylight hour until appearing a photosensitivity related symptom
   (b) a timing of the photosensitivity related symptom
   (c) a grade of the photosensitivity related symptom
   (d) PGIC (Patient Global Impression of Change) score
   (e) a skin pigment information
   (f) a melanin density
   (g) a quantity of a protoporphyrin in a blood
   (h) a type of a medicine
   (i) a prescription quantity of a medicine
   (j) Fitzpatrick skin type
   (k) PGIS score (Patient Global Impression Severity)
   (l) a data acquired by PROMIS (Patient-Reported Outcomes Measurement Information System)

### [Item 2]

The information processing device according to item 1,
wherein the provision information is an information for prompting a light blocking.

### [Item 3]

The information processing device according to items 1 or 2,
wherein the provision information is, calculated from the reference information set, the information related to an estimation sunshine duration until appearing the photosensitivity related symptom.

### [Item 4]

The information processing device according to item 1 to 3,
further comprising: an information provision unit;
the generation unit generates the information related to a remaining time of the estimation sunshine duration;
wherein the information provision unit notifies the remaining time of the estimation sunshine duration to the terminal.

### [Item 5]

The information processing device according to any one of items 1 to 4,
wherein the environmental data includes one or more selected from a solar radiation data, a sunlight wavelength data, and a weather information.

### [Item 6]

The information processing device according to any one of items 1 to 5,
Wherein, the one or more reference information selected from (a) to (1) is acquired from a clinical trial information database.

### [Item 7]

The information processing device according to item 1 to 6,
wherein the reference information set further includes a self-reported information of a patient needs a treatment for the photosensitivity.

### [Item 8]

The information processing device according to item 1 to 7,
wherein the self-reported information includes one or more selected from below (Ia) to (Im).
(Ia) a self-reported information of the patient needs the treatment for phototoxic dermatitis
(Ib) the self-reported information of the patient needs the treatment for photoallergic dermatitis
(Ic) the self-reported information of the patient needs the treatment for pellagra
(Id) the self-reported information of the patient needs the treatment for porphyria
(Ie) the self-reported information of the patient needs the treatment for xeroderma pigmentosum
(If) the self-reported information of the patient needs the treatment for Cockayne syndrome
(Ig) the self-reported information of the patient needs the treatment for Bloom syndrome
(Ih) the self-reported information of the patient needs the treatment for albinism
(Ii) the self-reported information of the patient needs the treatment for phenylketonuria
(Ij) the self-reported information of the patient needs the treatment for hydroa vacciniforme
(Ik) the self-reported information of the patient needs the treatment for solar urticaria
(Il) the self-reported information of the patient needs the treatment for polymorphic light eruption
(Im) the self-reported information of the patient needs the treatment for chronic photodermatitis

### [Item 9]

The information processing device according to item 1 to 8,
wherein the (Id) the self-reported information of the patient needs the treatment for the porphyria includes one or more selected from below (Ida) to (Idf).
(Ida) the self-reported information of the patient needs the treatment for erythroblastic protoporphyria (EPP)
(Idb) the self-reported information of the patient needs the treatment for X-linked porphyria (XLP)
(Idc) the self-reported information of the patient needs the treatment for congenital erythropoietic porphyria (CEP)
(Idd) the self-reported information of the patient needs the treatment for variegate porphyria (VP)
(Ide) the self-reported information of the patient needs the treatment for acute intermittent porphyria (AIP)
(Idf) the self-reported information of the patient needs the treatment for porphyria cutanea tarda (PCT)

### [Item 10]

The information processing device according to any one of items 1 to 9,
wherein the self-reported information is acquired from the terminal owned by the patient.

### [Item 11]

The information processing device according to any one of items 1 to 10,
wherein the reference information set is updated based on the self-reported information.

### [Item 12]

The information processing device according to any one of items 1 to 11,
wherein the information provision unit provides the provision information to the terminal owned by the patient or a doctor terminal operable by a doctor.

### [Item 13]

The information processing device according to item 1 to 12,
wherein the acquisition unit acquires an input information from the doctor terminal.

### [Item 14]

The information processing device according to any one of items 1 to 13,
wherein the acquisition unit acquires one or more data selected from (x) a solar radiation quantity, (y) an intensity distribution of a light spectrum, and (z) a going out time of the patient.

### [Item 15]

An information processing system comprising:
the acquisition unit acquires the reference information set including one or more reference information selected from below the (a) to (1), in addition the environmental data; and
the generation unit generates, based on the reference information set and the environmental data, the provision information for providing to the predetermined terminal;
   (a) the daylight hour until appearing the photosensitivity related symptom
   (b) the timing of the photosensitivity related symptom
   (c) the grade of the photosensitivity related symptom
   (d) the PGIC (Patient Global Impression of Change) score
   (e) the skin pigment information
   (f) the melanin density
   (g) the quantity of the protoporphyrin in the blood
   (h) the type of the medicine
   (i) the prescription quantity of the medicine
   (j) the Fitzpatrick skin type
   (k) the PGIS score (Patient Global Impression Severity)
   (l) the data acquired by the PROMIS (Patient-Reported Outcomes Measurement Information System)

### [Item 16]

An information processing method using a computer,
the information processing method comprising:
A step of acquiring the reference information set including one or more reference information selected from below the (a) to (l), in addition the environmental data; and
a step of generating, based on the reference information set and the environmental data, the provision information for providing to the predetermined terminal;
   (a) the daylight hour until appearing the photosensitivity related symptom
   (b) the timing of the photosensitivity related symptom
   (c) the grade of the photosensitivity related symptom
   (d) the PGIC (Patient Global Impression of Change) score
   (e) the skin pigment information
   (f) the melanin density
   (g) the quantity of the protoporphyrin in the blood
   (h) the type of the medicine
   (i) the prescription quantity of the medicine
   (j) the Fitzpatrick skin type
   (k) the PGIS score (Patient Global Impression Severity)
   (l) the data acquired by the PROMIS (Patient-Reported Outcomes Measurement Information System)

### [Item 17]

A program for executing the information processing method to the computer,
the program, as the information processing method, comprising:
the step of acquiring the reference information set including one or more reference information selected from below the (a) to (1), in addition the environmental data; and
the step of generating, based on the reference information set and the environmental data, the provision information for providing to the predetermined terminal;
   (a) the daylight hour until appearing the photosensitivity related symptom
   (b) the timing of the photosensitivity related symptom
   (c) the grade of the photosensitivity related symptom
   (d) the PGIC (Patient Global Impression of Change) score
   (e) the skin pigment information
   (f) the melanin density
   (g) the quantity of the protoporphyrin in the blood
   (h) the type of the medicine
   (i) the prescription quantity of the medicine
   (j) the Fitzpatrick skin type
   (k) the PGIS score (Patient Global Impression Severity)
   (l) the data acquired by the PROMIS (Patient-Reported Outcomes Measurement Information System)

### <Details of the embodiment>

A specific instance of an information processing device 10 according to a one instance of the embodiment of the present invention will be described with reference to drawings.

In the present embodiment, as described in "Hiroshi Shimizu, 'Atarashii Hifuka kagaku 3rd Edition' , Nakayama Shoten, 2018," "a photosensitivity" is a synonym with "a photosensitive dermatitis" and is a general term for skin diseases that are caused or exacerbated by exposure to a sunlight or an ultraviolet ray. Therefore, in this embodiment, the photosensitivity can include all of skin disease that is caused or exacerbated by exposure to the sunlight or the ultraviolet ray. For instance, the photosensitivity includes phototoxic dermatitis, photoallergic dermatitis, pellagra, porphyria, xeroderma pigmentosum, Cockayne syndrome, Bloom syndrome, albinism, phenylketonuria, hydroa vacciniforme, solar urticaria, polymorphic light eruption, chronic photodermatitis or the like.

One of a symptom of the photosensitivity, "porphyria" , is a disease appearing due to accumulation of a porphyrin bodies or their precursors by decreased activity of a heme-metabolizing enzymes, and one of the symptoms is the photosensitivity (sunburn, burn-like symptom). For instance, the porphyria includes erythropoietic protoporphyria (EPP), X-linked protoporphyria (XLP), congenital erythropoietic porphyria (CEP), variegate porphyria (VP), and acute intermittent porphyria (AIP), porphyria cutanea tarda (PCT), or the like.

"The photosensitivity related symptom" means a state that the photosensitivity patient is appeared or exacerbated by expose a sunlight or an ultraviolet ray, and a prodromal symptom (burning, stinging, itching or stinging) changes to a pain after a sunlight exposure. Therefore, in this embodiment, the photosensitivity related symptom includes both the prodromal symptoms and the pain.

### <Configuration>

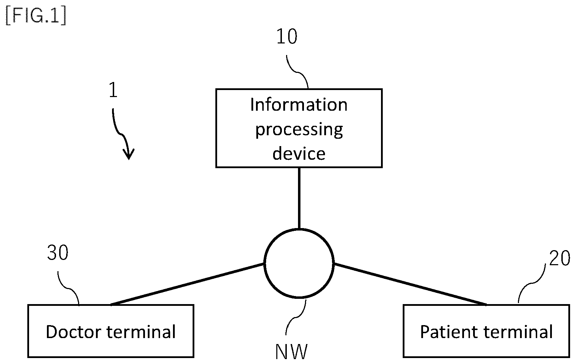

FIG. 1 is a diagram showing instance of an information processing system 1 including the information processing device 10 according to this embodiment. In this embodiment, the information processing device 10 is communicably connected to a patient terminal 20 owned by a patient and a doctor terminal 30 via a network NW.

In this embodiment, the network NW is, for instance, the Internet. The network NW is constructed by, for instance, a public telephone line network, a mobile phone line network, a wireless communication network, an Ethernet (registered trademark), or the like.

The information processing device 10 is a terminal managed by a medical institution or an organization that provides a medical information, and by executing to processing the information via the patient terminal 20, the doctor terminal 30 and the network NW, and is configured to a part of the information provision system 1. The information processing device 10 may be, for instance, a workstation, a general-purpose computer such as a personal computer, or logically realized by a cloud computing. The information processing device 10 may be installed an application capable of communicating with the patient terminal 20 or the doctor terminal 30, or installed a browser for accessing a web service that enables a communication.

The patient terminal 20 executes to processing the information via the information processing device 10, the doctor terminal 30 and the network NW. The information processing device 10 may be, for instance, the general-purpose computer such as the workstation or the personal computer, or may be the mobile communication device such as a smart phone or the like. In addition, the patient terminal 20 may be a wearable device worn by the patient oneself. The patient terminal 20 may be installed the application capable of communicating with the information processing device 10 or the doctor terminal 30, or installed the browser for accessing the web service that enables the communication.

The doctor terminal 30 is, for instance, the terminal for grasping a condition of the patient, the terminal used by a doctor working in a medical institution such as a hospital, and executes the information processing via the information processing device 10 or the patient terminal 20 in addition the network NW. The doctor terminal 30 may be, for instance, the workstation or the general-purpose computer such as the personal computer, or may be the mobile communication device such as the smart phone. The doctor terminal 30 may be installed the application capable of communicating with the information processing device 10 or the patient terminal 20, or installed the browser for accessing the web service that enables the communication.

### <Hardware configuration>

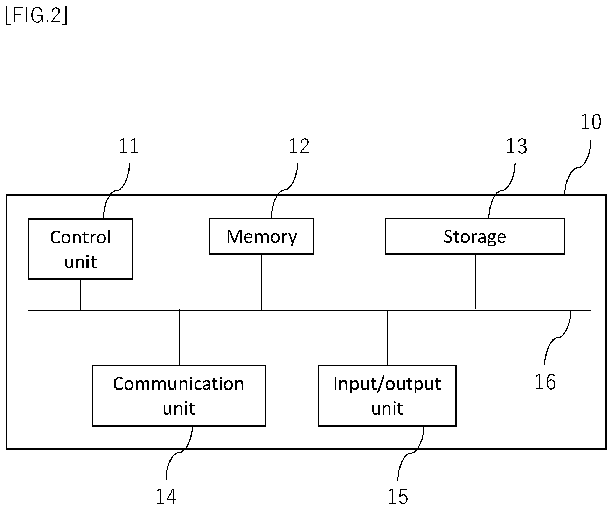

FIG. 2 is a diagram showing a hardware configuration instance of a computer that implements the information processing device 10 according to the present embodiment. The computer includes at least a control unit 11, a memory 12, a storage 13, a communication unit 14, and an input/output unit 15, or the like. These are electrically connected to each other by a bus 16.

The control unit 11 controls an overall operation of the information processing device 10, and is an arithmetic device executing the information processing necessary for controlling transmission and reception of a data and authentication processing and executing the application on between elements. For instance, the control unit 11 is a processor such as a CPU (Central Processing Unit) or the like, and by executing a program or the like stored in the storage 13 and read in the memory 12, executes each the information processing.

The memory 12 includes a main memory configurated to a volatile memory device such as a DRAM (Dynamic Random Access Memory) or the like, and an auxiliary memory device configurated to a non-volatile memory device such as a flash memory or an HDD (Hard Disc Drive) or the like. The memory 12 is used as a work area or the like for the control unit 11, and stores executed a BIOS (Basic Input/Output System) when the information processing device 10 is starting, and various setting information or the like.

The storage 13 stores various the programs such as application programs or the like. In the storage 13 may be constructed a database storing the data that used for each processing.

The communication unit 14 connects the information processing device 10 to the network. The communication unit 14 uses, for instance, by a wired LAN (Local Area Network), a wireless LAN, a Wi-Fi (Wireless Fidelity, registered trademark), an infrared communication, a Bluetooth (registered trademark), a short-distance or a contactless communication, or the like, communicates with an external device directly or via a network access point.

The input/output unit 15 is, for instance, an information input device such as a keyboard, a mouse, or a touch panel or the like, and output devices such as a display or the like.

The bus 16 is commonly connected to each of the above elements, and transmits, for instance, an address signal, a data signal and various a control signal or the like.

In the present embodiment, the hardware configuration of the terminal such as the computers and the smartphones or the like implement the patient terminal 20 and the doctor terminal 30, is the same as the hardware configuration instance of the information processing device 10 shown in FIG. 2, therefore, a description is omitted.

### <Software configuration>

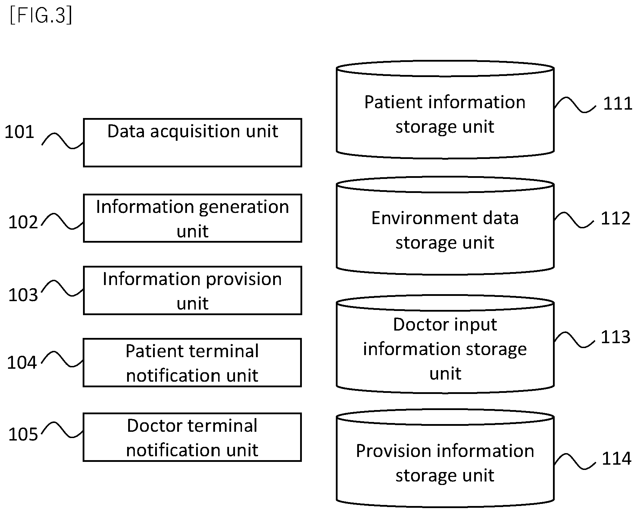

FIG. 3 is a diagram showing a software configuration instance of the information processing device 10 according to the present embodiment. The information processing device 10 can comprise each functional units such as a data acquisition unit 101, an information generation unit 102, an information provision unit 103, a patient terminal notification unit 104 a and doctor terminal notification unit 105, in addition, each storage units such as a patient information storage unit 111, an environment data storage unit 112, a doctor input information storage unit 113, and a provision information storage unit 114.

In addition, the data acquisition unit 101, the information generation unit 102, the information provision unit 103, the patient terminal notification unit 104, and the hospital terminal notification unit 105 are implemented, due to the program stored in the storage 13 are read and executed to the memory 12 by the control unit 11 that comprised the information processing device 10. The patient information storage unit 111, the environment data storage unit 112, the doctor input information storage unit 113 and the provision information storage unit 114 are implemented as a part of storage areas that provided by at least one of the memory 12 or the storage 13.

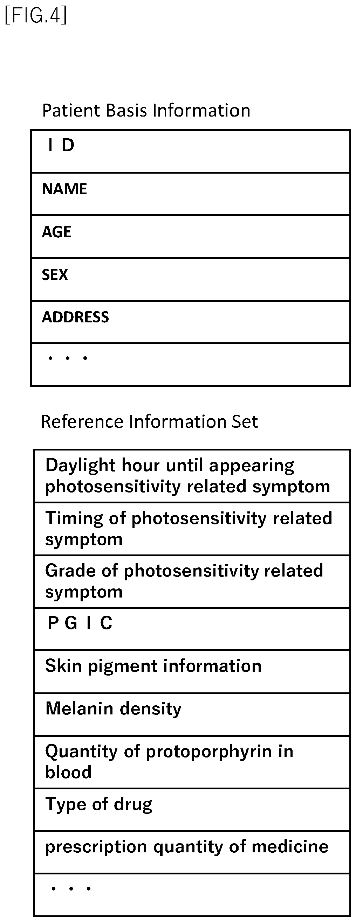

The patient information storage unit 111 stores the patient information acquired by the data acquisition unit 101. The patient information storage unit 111 includes, for instance, a patient basic information and a patient reference information set. FIG. 4 is the configuration instance of the patient information stored in the patient information storage unit 111. The Patient information may be related to a patient ID. The patient basic information includes, related to the patient, the information related to attributes such as a name, an age, a gender, an address, a profession, a hometown or the like, and an information related to lifestyles such as a chronic disease, a medical history, an allergy, a constitution (obesity, weakness, or the like), an eating habit, a drinking, a smoking, and an exercise habit or the like.

The patient reference information set stored in the patient information storage unit 111 includes, for instance, one or more the patient reference information selected from below (a) to (1).
(a) a daylight hour until appearing a photosensitivity related symptom
(b) a timing of the photosensitivity related symptom
(c) a grade of the photosensitivity related symptom
(d) PGIC (Patient Global Impression of Change) score
(e) a skin pigment information
(f) a melanin density
(g) a quantity of a protoporphyrin in a blood
(h) a type of a medicine
(i) a prescription quantity of a medicine
(j) Fitzpatrick skin type
(k) PGIS score (Patient Global Impression Severity)
(l) a data acquired by PROMIS (Patient-Reported Outcomes Measurement Information System)

These the reference information set may be, for instance, the information acquired from database (not shown) that stores data (a clinical trial data) acquired from a clinical trial conducted at medical institutions or the like, and may be a self-reported information of the patient needs treatment for the photosensitivity, for instance, erythropoietic protoporphyria (EPP) and/or X-linked porphyria (XLP).

When the reference information set is the information acquired from the clinical trial information database, the reference information set may include, in addition to the above (a) to (l), a date of conducting the clinical trial, the date of data acquisition. The self-reported information may be information directly input to the patient terminal 20 by the patient using the patient terminal 20. In addition, the reference information set may be information acquired from the database (not shown) storing information input by a doctor that examined patient uses the doctor terminal 30. These reference information sets may be updated based on the self-reported information.

(a) " a daylight hour until appearing a photosensitivity related symptom" is, for instance, in past the clinical trial, a sunlight or ultraviolet exposure time until appearing the photosensitivity related symptom to the photosensitivity patient. For instance, it means time until appearing the photosensitivity related symptom in the clinical trial of a compound A for the patient with erythroblastic protoporphyria and the X-linked porphyria. That is, in a randomized double blind clinical trial that adult male and female patients with the erythroblastic protoporphyria and the X-linked porphyria are medicated to the compound A for 16 weeks, it means, between from 1 hour after sunrise to 1 hour before sunset, the time until first appearing the symptoms due to light exposure by not only a direct sunlight but also an indirect sunlight.
   Here, the compound A is, for instance, a co-crystal including 1-{2-[(3S,4R)-1-{[(3R,4R)-1-cyclopentyl-3-fluoro-4-(4-methoxyphenyl) pyrrolidine-3-yl] carbonyl}-4-(methoxymethyl) pyrrolidin-3-yl]-5-(trifluoromethyl) phenyl} piperidine-4-carboxylic acid and phosphoric acid.
(b) "a timing of the photosensitivity related symptom" is the number of the photosensitivity related symptom recorded by the photosensitivity patient. For instance, it means, in the clinical trials of the compound A for the patient with the erythroblastic protoporphyria and the patient with the X-linked porphyria, the number of the symptom related to photosensitivity events recorded by the patient during the 16-week evaluation period.
(c) "a grade of the photosensitivity related symptom" is, about an intensity (degree) and a pattern of the photosensitivity related symptom recorded by the photosensitivity patient, a step-by-step evaluation using the number or the like. For instance, the pain is divided into 11 levels ranging from 0 to 10, no the pain at all is 0 and the worst the pain imaginable is 10, a degree of the pain is represented by a score.
(d) "PGIC (Patient Global Impression of Change) score" means health-related QOL of the patient. For instance, it means, the health-related QOL evaluation of the patient in the clinical trial of the compound A for the patient with the erythroblastic protoporphyria and the patient with the X-linked porphyria, and it means, recorded by patient oneself at week 16 of the clinical trial, an improvement degree in an overall health condition including a physical and a mental. For instance, in a questionnaire that evaluates the improvement degree on a 7-point scale, the lowest score is 7 points, it indicates "remarkably worsened", and the highest score is 1 point, it indicates "remarkably improved".
(e) "a skin pigmentation information" means the skin pigmentation information of the photosensitivity patient.
(f) "a melanin density" means the melanin density of the skin of the photosensitivity patient. For instance, it means, by using a spectrophotometer at the 16 weeks of the clinical trial, the melanin density acquired by measuring the six skins of the patient that a forehead, a left cheek, a right inner upper arm, a left inner outer arm, a right abdomen, and a left buttock.
(g) " a quantity of a protoporphyrin in a blood" is the quantity of the protoporphyrin in the blood of the photosensitivity patient. For instance, the quantity of the protoporphyrin in the blood of the photosensitivity patient at the 16 weeks of the clinical trial.
(h) "a type of a medicine" means the type of the medicine medicated to the photosensitivity patient. For instance, it means the type of the medicine medicated in the clinical trial.
(i) "a prescription quantity of a medicine" means the prescribed quantity of the medicine medicated to the photosensitivity patient. For instance, it means the prescription quantity of the medicine medicated in the clinical trial. Here, the medication period of the medicine may be included.
(j) "Fitzpatrick skin type" is, based on a sensitivity by exposure to the ultraviolet ray, a scale that classifies a human skin color into six stages (type 1 to type 6). In particular, it is a scale classified according to how to appear a "red sunburn" and a "black sunburn". The "red sunburn" is a burn that appears when expose to an ultraviolet light. The "black sunburn" is, as a reaction after the "red sunburn", appearing by synthesizing melanin in the skin to protect a human body from the ultraviolet ray. For instance, it means the skin type of the skin in the above clinical trials.
(k) "PGIS score (Patient Global Impression Severity)" is an overall severity degree that may be used to evaluate a symptom degree of a disease by an impression of the patient. For instance, in the clinical trial, the overall severity degree is evaluated by using a score given by a subject. The subject means evaluating on a 5-point scale from 'none' to 'very severe' about the severity degree recognized by the subject oneself.
(l) "a data acquired by PROMIS (Patient-Reported Outcomes Measurement Information System)" is the data acquired from indices evaluating seven domains that a physical function, an anxiety, a depression, a fatigue, a sleep disturbance, an ability that participate in social roles and activities, and a disability due to the pain (including pain intensity). For instance, it means a change quantity of the PROMIS score (total score and total score of each the domains) in the above clinical trial.

The above (a) to (1) may be updated based on the self-reported information.

The self-reported information includes, for instance, one or more selected from below (Ia) to (Im).
(Ia) the self-reported information of the patient needs the treatment for phototoxic dermatitis
(Ib) the self-reported information of the patient needs the treatment for photoallergic dermatitis
(Ic) the self-reported information of the patient needs the treatment for pellagra
(Id) the self-reported information of the patient needs the treatment for porphyria
(Ie) the self-reported information of the patient needs the treatment for xeroderma pigmentosum
(If) the self-reported information of the patient needs the treatment for Cockayne syndrome
(Ig) the self-reported information of the patient needs the treatment for Bloom syndrome
(Ih) the self-reported information of the patient needs the treatment for albinism
(Ii) the self-reported information of the patient needs the treatment for phenylketonuria
(Ij) the self-reported information of the patient needs the treatment for hydroa vacciniforme
(Ik) the self-reported information of the patient needs the treatment for solar urticaria
(Il) the self-reported information of the patient needs the treatment for polymorphic light eruption
(Im) the self-reported information of the patient needs the treatment for chronic photodermatitis

Above (Id) the self-reported information of the patient needs the treatment for porphyria includes, for instance, one or more selected from below (Ida) to (Idf).
(Ida) The self-reported information of the patient needs the treatment for erythroblastic protoporphyria (EPP)
(Idb) The self-reported information of the patient needs the treatment for X-linked porphyria (XLP)
(Idc) The self-reported information of the patient needs the treatment for congenital erythropoietic porphyria (CEP)
(Idd) The self-reported information of the patient needs the treatment for variegate porphyria (VP)
(Ide) The self-reported information of the patient needs the treatment for acute intermittent porphyria (AIP)
(Idf) The self-reported information of the patient needs the treatment for porphyria cutanea tarda (PCT)

The above self-reported information may be updated each time the self-reported information is acquired.

The reference information set of the patient that stored in the patient information storage unit 111 may include the information on a regular medicine or the like that a medicine of other than the photosensitivity, in that case, it may be included those the prescribed quantity. In addition, it may be included an image of the skin photographed by using a camera or the like.

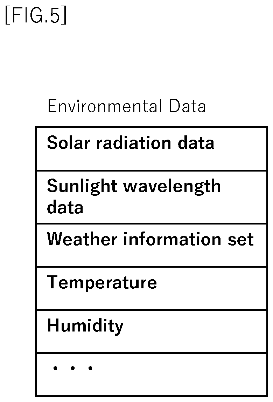

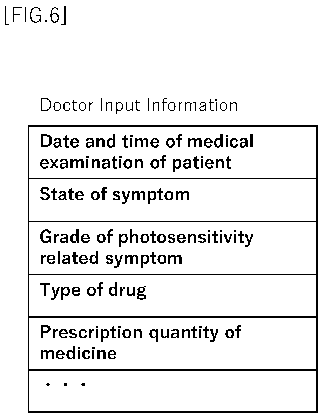

The environment data storage unit 112 stores the data related to the environment acquired by the data acquisition unit 101. FIG. 5 is the configuration instance of the environmental data stored in the environmental data storage unit 112. The environmental data may include, for instance, a solar radiation quantity data such as the quantity of an ultraviolet or the like, a sunlight wavelength data, and a weather information, in addition it may also include a temperature, a humidity, a wind speed, or the like. These information may be the data automatically or manually acquired from a solar radiation quantity measurement application or a sunlight measurement application installed in the information processing device 10, and it may acquire the data input to the patient terminal 20 by the patient. When the patient terminal 20 is a wearable device terminal, the environmental data may be the data directly acquired by the wearable device terminal. And, it may automatically acquire weather the information of a specific area disclosed by the Meteorological Agency or the like. That is, it may be, on cooperation with a publicly known information, the information acquired automatically and stored by the information processing device 10. The doctor input information storage unit 113 stores the doctor input information acquired from the doctor terminal 30. FIG. 6 is the configuration instance of the doctor input information stored in the doctor input information storage unit 113. The doctor input information includes, for instance, a medical examination information of the patient, in other words, the date and time of the medical examination information of the patient and the same information as the (a) to (1) included in the above reference information set.

The doctor input information storage unit 113 stores the doctor input information acquired from the doctor terminal 30. FIG. 6 is a configuration instance of the doctor input information stored in the doctor input information storage unit 113. The doctor input information includes, for instance, the medical examination information of the patient, that is, the date and time of the medical examination of the patient, and the information similar to (a) to (1) included in the above reference information set.

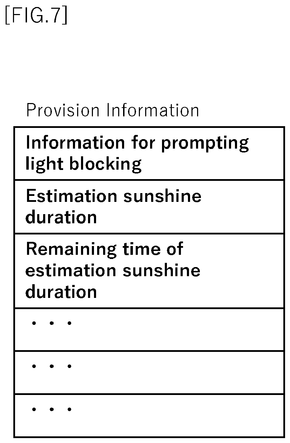

The provision information storage unit 114 stores the provision information generated based on both the reference information set stored in the patient information storage unit 111 and the environment data stored in the environment data storage unit 112. FIG. 7 is the configuration instance of the provision information stored in the provision information storage unit 114. The provision information is, for instance, the information for prompting the light blocking, the estimation sunshine duration until appearing the photosensitivity related symptom calculated from the reference information set, and a remaining time until appearing the photosensitivity related symptom.

The data acquisition unit 101 acquires the reference information set including one or more reference information selected from the (a) to (1), in addition the environmental data. The data acquisition unit 101 may acquire the reference information set including the reference information and the environmental data from in the patient terminal 20 or in the doctor terminal 30, or via another data server. And, it may acquire, from an application installed in the information processing device 10, for instance, the information of a solar radiation quantity, or an intensity distribution of a light spectrum, the information of a going out time of the patient. Also, the data acquisition unit 101 may accept, about the data related to the reference information of a patient oneself in addition the environmental data, for instance, an input of the data input by the patient oneself, the input of the data by a person other than the patient such as family and friends of the patient, or the input of the data by more than one the person. The information acquired by the data acquisition unit 101 is stored in the patient information storage unit 111, the environmental data storage unit 112, or the doctor input information storage unit 113, respectively.

The information generation unit 102 generates, based on the data acquired by the data acquisition unit 101, provision information provided to a predetermined terminal. That is, it generates, based on the reference information set of the patient including one or more reference information selected from the (a) to (1) in addition environmental data, the information provided to the patient and the doctor. The information provided to the patient is, as described above, for instance, the information for prompting the light blocking, the estimation sunshine duration until appearing the photosensitivity related symptom calculated from the reference information set, and the remaining time until appearing the photosensitivity related symptom. A generated information is transmitted to the patient terminal 20 and the doctor terminal 30 by the information provision unit 103. The information generated by the information generation unit 102 is stored in the provision information storage unit 114.

The information provision unit 103 provides the provision information generated by the information generation unit 102 to the predetermined terminal. A terminal provided the information by the information providing unit 103 may be the patient terminal 20 owned by the patient, the doctor terminal 30, or other a third party's terminal.

The patient terminal notification unit 104 notifies, for instance, at a prescribed timing, a message prompting to acquire the reference information or the environmental data to the patient. The timing may be set for each the patient, and can be set intervals and time periods, for instance, notifying at a predetermined time every day, notifying once a week, or the like. In addition, based on the provision information notified from the information provision unit 103, it may issue a warning. For instance, the timing that the remaining time of the estimation sunshine duration is short, for instance, at the timing of the remaining time is less than 1 hour, it may issue the warning for indicating a high possibility will be appearing photosensitivity related symptom.

The doctor terminal notification unit 105 notifies, for instance, in case of acquiring the provision information of the patient from the information provision unit 103 at the predetermined timing, a message for prompting to confirm the provision information. In addition, for instance, in case of input an appointment for an appointment for a medical examination from the patient terminal 20 and the appointment information is acquired via the information processing device 10, it may notify the appointment information.

### <Flow of information processing>

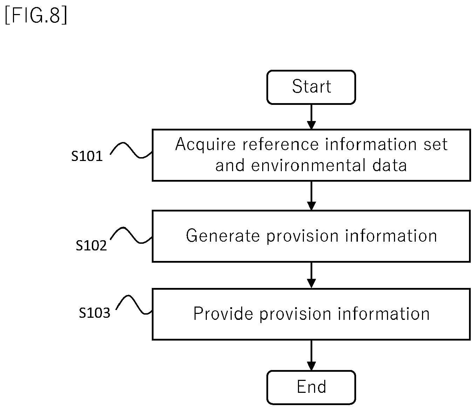

FIG. 8 is a diagram showing flow of processing executed in the information processing device 10 according to a first embodiment.

First, as a preprocessing for this processing, the data acquisition unit 101 of the information processing device 10 accepts the input of the information related to the patient and stores the patient basic information in the patient information storage unit 111. When the patient information is already stored in the patient information storage unit 111, by accepting the input of a patient ID or the like, it is possible to refer to the patient information necessary for the information processing according to the present embodiment.

Next, the data acquisition unit 101 acquires the reference information set including one or more the reference information selected from the above (a) to (1), in addition the environmental data (S101). About acquiring of these data, it may be acquired by acquiring the data held by the patient terminal 20, or the doctor terminal 30, or another server, or it may be automatically acquired by the information processing device 10 in cooperation with the publicly known information, or it may be acquired by an installed application. For instance, when the solar radiation quantity data or the sunlight wavelength data are acquired, the corresponding application is activated and acquire the data.

The information generation unit 102 generates, based on the data acquired by the data acquisition unit 101, the provision information to be provided to the predetermined terminal (S102). That is, based on the patient reference information set including one or more the reference information selected from the (a) to (1) in addition the environmental data, it generates the information provided to the patient, the doctor, or the like.

The information provision unit 103 provides the provision information generated by the information generation unit 102 to the predetermined terminal (S103). The terminal that the information provided by the information provision unit 103 may be the patient terminal 20 owned by the patient, the doctor terminal 30, or a third party's terminal. The information provided to the patient terminal 20 is, for instance, the information for prompting the light blocking, the estimation sunshine duration until appearing the photosensitivity related symptom, and the remaining time until appearing the photosensitivity related symptom.

In this way, the information processing device 10 of the present embodiment predicts the timing appearing the photosensitivity related symptom to the photosensitivity patient, and provides a result to the predetermined terminal. As a result, the photosensitivity patient can know the timing appearing the photosensitivity related symptom by conforming the provision information provided to the patient terminal 20, hereby, by blocking a light or changing the time of going out, it is possible to prevent from appearing the photosensitivity related symptom. When there is the remaining time until appearing the photosensitivity related symptom, it is possible to go out without blocking the light.

Therefore, according to the information processing device 10 of the present embodiment, by the photosensitivity patient knows the timing appearing the photosensitivity related symptom in advance, not avoiding to go out by fearing about appearing the photosensitivity related symptom, QOL improves by adjusting time.

Although the present embodiment has been described above, the above embodiment is intended to understand of the present invention easier, and is not intended to limit to interpret the present invention. The present invention may be modified and improved without departing from its spirit, and the present invention also includes equivalents thereof.

For instance, in the present embodiment, for convenience of an explanation, the information processing device 10, the patient terminal 20, and the doctor terminal 30 are illustrated one each, however a plurality of the patient terminal 20 and the doctor terminal 30 can connect to the information processing device 10 via the network NW. Further, although the information processing device 10 is estimated to be one computer, it is not limited to this, a system may be configured to functional units and storage union that are distributed to a plurality of computers. For instance, each storage unit of the information processing device 10 may comprise in a database server, and the information processing device 10 may access the database server. In addition, it can comprise each functional units are distributed to the plurality of computers.

In addition, the information processing device 10 is the patient terminal owned by the patient, and may be configured to access separately provided the database server.

Further, it may be included configurations other than the functional units and configuration the units included in FIG. 3. In addition, it may be added that steps other than each the steps included in FIG. 8. For instance, in S101, when acquiring the skin image of the patient captured by a camera (not shown), an image acquisition application may be activated and acquire the skin image. In addition, in S101, the reference information set may be updated based on the self-reported information. This makes it possible to predict the timing of appearing the photosensitivity related symptom to the photosensitivity patient more accurately.

In addition, in S103 or after S103, based on the provision information, the warning may be issued to the patient terminal 20. Further, based on the provision information, it may comprise an evaluation unit that evaluates a medicine efficacy in the clinical trials, and may notify the result to the doctor terminal 30. In addition, based on the evaluation, it may be set to prompt the medical examination to the patient. In that case, it may receive a reservation of the medical examination, and may notify of the reservation of the medical examination to the doctor terminal 30.

Above, with reference to the accompanying drawings, a preferred embodiment of the present disclosure has been described in detail, however, the technical scope of the present disclosure is not limited to such instances. It is obvious that a person ordinarily skilled in the art of the present disclosure can conceive various modifications or modifications within the scope of a technical idea described in claims, these are naturally included within a technical scope of the present disclosure.

In addition, an effect described in this description are merely for describing or explaining, and are not limiting. In other words, a technique according to the present disclosure can produce, in addition to above effects or instead of the above effects, can occur other the effects that are obvious the effects for the person ordinarily skilled in the art.

### [description of the reference numerals]

- 1: information processing system
- 10: information processing device
- 11: control unit
- 12: memory
- 13: storage
- 14: communication unit
- 15: input/output unit
- 16: bus
- 20: patient terminal
- 30: doctor terminal
- 101: data acquisition unit
- 102: information generation unit
- 103: information provision unit
- 104: patient terminal notification unit
- 105: doctor terminal notification unit
- 111: patient information storage unit
- 112: environment data storage unit
- 113: doctor input information storage unit
- 114: provision information storage unit
- NW: network

## Claims

1. An information processing device comprising:
an acquisition unit acquires a reference information set including one or more reference information selected from below (a) to (1), in addition an environmental data; and
a generation unit generates, based on the reference information set and the environmental data, a provision information for providing to a predetermined terminal;
(a) a daylight hour until appearing a photosensitivity related symptom
(b) a timing of the photosensitivity related symptom
(c) a grade of the photosensitivity related symptom
(d) PGIC (Patient Global Impression of Change) score
(e) a skin pigment information
(f) a melanin density
(g) a quantity of a protoporphyrin in a blood
(h) a type of a medicine
(i) a prescription quantity of a medicine
(j) Fitzpatrick skin type
(k) PGIS score (Patient Global Impression Severity)
(l) a data acquired by PROMIS (Patient-Reported Outcomes Measurement Information System)

2. The information processing device according to claim 1,
wherein the provision information is an information for prompting a light blocking.

3. The information processing device according to claims 1 or 2,
wherein the provision information is, calculated from the reference information set, the information related to an estimation sunshine duration until appearing the photosensitivity related symptom.

4. The information processing device according to claim 3,
further comprising: an information provision unit;
the generation unit generates the information related to a remaining time of the estimated sunshine duration;
wherein the information provision unit notifies the remaining time of the estimation sunshine duration to the terminal.

5. The information processing device according to any one of claims 1 to 4,
wherein the environmental data includes one or more selected from a solar radiation data, a sunlight wavelength data, and a weather information.

6. The information processing device according to any one of claims 1 to 5,
Wherein, the one or more reference information selected from (a) to (1) is acquired from a clinical trial information database.

7. The information processing device according to claim 6,
wherein the reference information set further includes a self-reported information of a patient needs a treatment for the photosensitivity.

8. The information processing device according to claim 7,
wherein the self-reported information includes one or more selected from below (Ia) to (Im).
(Ia) a self-reported information of the patient needs the treatment for phototoxic dermatitis
(Ib) the self-reported information of the patient needs the treatment for photoallergic dermatitis
(Ic) the self-reported information of the patient needs the treatment for pellagra
(Id) the self-reported information of the patient needs the treatment for porphyria
(Ie) the self-reported information of the patient needs the treatment for xeroderma pigmentosum
(If) the self-reported information of the patient needs the treatment for Cockayne syndrome
(Ig) the self-reported information of the patient needs the treatment for Bloom syndrome
(Ih) the self-reported information of the patient needs the treatment for albinism
(Ii) the self-reported information of the patient needs the treatment for phenylketonuria
(Ij) the self-reported information of the patient needs the treatment for hydroa vacciniforme
(Ik) the self-reported information of the patient needs the treatment for solar urticaria
(Il) the self-reported information of the patient needs the treatment for polymorphic light eruption
(Im) the self-reported information of the patient needs the treatment for chronic photodermatitis

9. The information processing device according to claim 8,
wherein the (Id) the self-reported information of the patient needs the treatment for the porphyria includes one or more selected from below (Ida) to (Idf).
(Ida) the self-reported information of the patient needs the treatment for erythroblastic protoporphyria (EPP)
(Idb) the self-reported information of the patient needs the treatment for X-linked porphyria (XLP)
(Idc) the self-reported information of the patient needs the treatment for congenital erythropoietic porphyria (CEP)
(Idd) the self-reported information of the patient needs the treatment for variegate porphyria (VP)
(Ide) the self-reported information of the patient needs the treatment for acute intermittent porphyria (AIP)
(Idf) the self-reported information of the patient needs the treatment for porphyria cutanea tarda (PCT)

10. The information processing device according to any one of claims 7 to 9,
wherein the self-reported information is acquired from the terminal owned by the patient.

11. The information processing device according to any one of claims 7 to 10,
wherein the reference information set is updated based on the self-reported information.

12. The information processing device according to any one of claims 1 to 11,
wherein the information provision unit provides the provision information to the terminal owned by the patient or a doctor terminal operable by a doctor.

13. The information processing device according to claim 12,
wherein the acquisition unit acquires an input information from the doctor terminal.

14. The information processing device according to any one of claims 1 to 13,
wherein the acquisition unit acquires one or more data selected from (x) a solar radiation quantity, (y) an intensity distribution of a light spectrum, and (z) a going out time of the patient.

15. An information processing system comprising:
the acquisition unit acquires the reference information set including one or more reference information selected from below the (a) to (1), in addition the environmental data; and
the generation unit generates, based on the reference information set and the environmental data, the provision information for providing to the predetermined terminal;
(a) the daylight hour until appearing the photosensitivity related symptom
(b) the timing of the photosensitivity related symptom
(c) the grade of the photosensitivity related symptom
(d) the PGIC (Patient Global Impression of Change) score
(e) the skin pigment information
(f) the melanin density
(g) the quantity of the protoporphyrin in the blood
(h) the type of the medicine
(i) the prescription quantity of the medicine
(j) the Fitzpatrick skin type
(k) the PGIS score (Patient Global Impression Severity)
(l) the data acquired by the PROMIS (Patient-Reported Outcomes Measurement Information System)

16. An information processing method using a computer,
the information processing method comprising:
a step of acquiring the reference information set including one or more reference information selected from below the (a) to (l), in addition the environmental data; and
a step of generating, based on the reference information set and the environmental data, the provision information for providing to the predetermined terminal;
(a) the daylight hour until appearing the photosensitivity related symptom
(b) the timing of the photosensitivity related symptom
(c) the grade of the photosensitivity related symptom
(d) the PGIC (Patient Global Impression of Change) score
(e) the skin pigment information
(f) the melanin density
(g) the quantity of the protoporphyrin in the blood
(h) the type of the medicine
(i) the prescription quantity of the medicine
(j) the Fitzpatrick skin type
(k) the PGIS score (Patient Global Impression Severity)
(l) the data acquired by the PROMIS (Patient-Reported Outcomes Measurement Information System)

17. A program for executing the information processing method to the computer,
the program, as the information processing method, comprising:
the step of acquiring the reference information set including one or more reference information selected from below the (a) to (l), in addition the environmental data; and
the step of generating, based on the reference information set and the environmental data, the provision information for providing to the predetermined terminal;
(a) the daylight hour until appearing the photosensitivity related symptom
(b) the timing of the photosensitivity related symptom
(c) the grade of the photosensitivity related symptom
(d) the PGIC (Patient Global Impression of Change) score
(e) the skin pigment information
(f) the melanin density
(g) the quantity of the protoporphyrin in the blood
(h) the type of the medicine
(i) the prescription quantity of the medicine
(j) the Fitzpatrick skin type
(k) the PGIS score (Patient Global Impression Severity)
(l) the data acquired by the PROMIS (Patient-Reported Outcomes Measurement Information System)
